# EUROPÄISCHE PATENTSCHRIFT

(11) **EP 0 998 897 B1**
(45) Veröffentlichungstag und Bekanntmachung des Hinweises auf die Patenterteilung: **28.02.2001**
(21) Anmeldenummer: 99118522.4
(22) Anmeldetag: 18.09.1999
(51) Int. Cl.: A61K 7/00, A61K 7/13

(54) **Verfahren zur Herstellung von stabilen wässrigen Haarfärbeemulsionen**
Method for manufacturing stable aqueous hair dye emulsions
Méthode de fabrication d'émulsions aqueuses stables pour la teinture des cheveux

(30) Priorität: 14.10.1998 DE 19847224
(43) Veröffentlichungstag der Anmeldung: 10.05.2000
(73) Patentinhaber: GOLDWELL AKTIENGESELLSCHAFT, D-64297 Darmstadt (DE)
(72) Erfinder: Golinski, Frank, Dr., 64297 Darmstadt (DE); Lorenz, Heribert, 64401 Gross-Bieberau (DE)

(56) Entgegenhaltungen:
- EP-A- 0 227 430
- DE-A- 3 834 142
- DE-A- 4 103 292
- DE-A- 4 219 981

## Beschreibung

Die vorliegende Erfindung betrifft ein verbessertes Verfahren zur Herstellung von stabilen wäßrigen Haarfärbeemulsionen.

Permanente Haarfärbemittel, die üblicherweise mindestens ein Oxidationsfarbstoff-Vorprodukt, nämlich ein Entwickler-Kuppler-System, enthalten, werden zumeist in Form wäßriger Emulsionen eingesetzt (vgl. K. Schrader, Grundlagen und Rezepturen der Kosmetika, 2. Aufl. (1989), S. 797 ff).

Deren Herstellung geschieht durch Heißemulgieren der Bestandteile und darauffolgendes Abkühlen, was naturgemäß eine längere Zeit in Anspruch nimmt und auch nicht immer zu stabilen Emulsionen führt.

Es bestand daher ein Bedürfnis, den gegenwärtigen Herstellungsprozeß zu ändern und dabei zu optimieren.

Es wurde nunmehr gefunden, daß eine stabile wäßrige Haarfärbeemulsion, die mindestens ein Oxidations-Haarfarbstoffvorprodukt enthält, mit guten rheologischen Eigenschaften unter wesentlicher Reduzierung der Herstellungszeit und entsprechender Energieersparnis dadurch hergestellt werden kann, daß eine Wasser-in-Öl-Emulsion, enthaltend 10 bis 50 Gew.-% mindestens eines nichtionischen Emulgators, 10 bis 50 Gew.-% mindestens eines nichtionischen Co-Emulgators, ausgewählt aus der Gruppe C₁₀-C₁₀-Fettalkohole, C₁₂-C₁₈-Fettsäuremono- und -dialkanolamide und/oder C₁₀-C₂₂-Fettsäureestern mit mehrwertigen Alkoholen, 5 bis 40 Gew.-% Ölsäure und bis zu 25, insbesondere 20 Gew.-% Wasser, jeweils berechnet auf die Gesamtzusammensetzung der W/0-Emulsion, mit einer wäßrigen Phase, die mindestens ein wasserlösliches Tensid enthält, unter Scherkraft, d.h. Rühren, bei 15 bis 30°C, vorzugsweise Raumtemperatur bei etwa 20 bis 25°C, vermischt wird.

Dabei weist das erhaltene Endprodukt vorzugsweise eine Viskosität zwischen 5000 und 30 000, insbesondere 7 500 und 25 000, besonders bevorzugt etwa 10 000 bis 20 000 mPa.s, gemessen bei 20°C im Brookfield-Viskosimeter RVT, auf.

Der Anteil der Ölphase in der Gesamtemulsion liegt dabei vorzugsweise bei etwa 5 bis etwa 40, vorzugsweise 10 bis 30 Gew.-% der Gesamtemulsion.

Die Wasser-in Öl-Emulsion kann auch noch etwa 0 bis 20 Gew.-% eines Öls enthalten.

Als Ölkörper in der Ölphase werden vorzugsweise die üblichen kosmetischen Öle und Fette, beispielsweise natürliche Öle wie Avocadoöl, Cocosöl, Palmöl, Sesamöl, Erdnußöl, Spermöl, Sonnenblumenöl, Mandelöl, Pfirsichkemöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline eingesetzt.
Synthetische Öle und Wachse sind beispielsweise Silikonöle, Polyethylenglykole, etc. Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Cetylpalmitat, Polyethylenglykol- und Polyglycerylfettsäureester wie PEG-7-glycerylcocoat, etc.

Geeignete nichtionische Emulgatoren sind insbesondere die verschiedenen C₁₀-C₂₂-Fettalkoholethoxylate wie Lauryl-, Myristyl-, Cetyl-, Oleyl-,Tridecyl-, Isotridecyl-, Cocosfett- und Talgfettalkoholethoxylate, etc; jedoch können auch weitere an sich bekannte nichtionische öllösliche Emulgatoren verwendet werden.

Die Zahl der Ethylenoxid-Moleküle pro Mol Fettalkohol liegt im Durchschnitt zwischen etwa 2 und 15, vorzugsweise etwa 4 bis 10.

Weitere geeignete nichtionische Emulgatoren sind C₈-C₁₈-Alkylpolyglucoside mit einem Kondensationsgrad von vorzugsweise 1,1 bis 3, insbesondere 1,2 bis 2,5, die für sich seit langem bekannt sind.
Auch weitere nichtionische Tenside, beispielsweise Aminoxide wie Lauryldimethylaminoxid, z.B. vom Typ "Ammonyx^{R}", "Aromax^{R}" oder "Genaminox^{R}" sind für diesen Zweck geeignet.

Geeignete nichtionische Emulgatoren für W/O-Emulsionen, deren Menge vorzugsweise bei etwa 15 bis 40, insbesondere etwa 20 bis 35 Gew.-%, berechnet auf die W/O-Emulsion beträgt, sind aus dem einschlägigen Stand der Technik, beispielsweise der Standardmonographie von K. Schrader, Grundlagen und Rezepturen der Kosmetika, 2. Aufl., S. 387-525 (1989), bekannt.

Der erfindungsgemäß in einer Menge von 10 bis 50 Gew.-%, vorzugsweise etwa 15 bis 4,5, insbesondere etwa 20 bis 40 Gew.-%, berechnet auf die W/O-Emulsion, eingesetzte Co-Emulgator, der auch verdickend und konsistenzregulierend wirkt, ist ausgewählt aus der Gruppe C₈-C₂₂-Fettalkohole, C₁₂-C₁₈-Fettsäuremonoalkanolamide und/oder C₁₀-C₂₂-Fettsäureestern mehrwertiger Alkohole.

Besonders bevorzugt sind hierbei von den Fettalkoholen Cocosfettalkohol, Laurylalkohol, Decylalkohol, Tridecylalkohol, Myristylalkohol, Cetylalkohol, Stearylalkohol und Gemische derselben.
Bevorzugte C₁₂-C₁₈-Fettsäurealkanolamide sind Cocamide MEA, Cocamide DEA, Cocamide MIPA, Lauramide MEA, Lauramide DEA, Oleamide MEA, Oleamide DEA, Stearamide MEA, Stearamide DEA und Stearamide MIPA.
Geeignete C₁₀-C₂₂-Fettsäureester mehrwertiger Alkohole sind insbesondere die Ethylenglykol-, Propylenglykol-, Polyethylenglykol-, Glycerin- und Sorbitanester von Laurinsäure, Cocosfettsäure, Myristinsäure oder Stearinsäure und Mischester derselben wie beispielsweise Polyethylenglykol (PEG-)-glycerylfettsäureester.

Diese Komponenten sind ebenfalls bekannt und bei Schrader, l.c., beschrieben.

Die W/O-Emulsion enthält als essentiellen Bestandteil schließlich noch Ölsäure in einer Menge von 5 bis 40, vorzugsweise etwa 10 bis 30, insbesondere etwa 15 bis 25 Gew.-% der Emulsion.

Ein weiterer bevorzugter Bestandteil in den W/O-Emulsionen ist Oleyalkohol, vorzugsweise in einer Menge von etwa 5 bis 15 Gew.-%, entweder als zusätzlicher Bestandteil oder auch als Fettalkohol-Bestandteil des Co-Emulgators.

Dieser Bestandteil dient insbesondere zur Verbesserung des ästhetischen Aussehens der Gesamtemulsion.

Der Wassergehalt der Wasser-in-Öl-Emulsion beträgt maximal 25 Gew.-% derselben.

Die Herstellung der W/O-Emulsion erfolgt zweckmäßigerweise durch Schmelzen der Fettphase-Komponenten bei etwa 50 bis etwa 80°C, Zugabe von Wasser und Abkühlen auf etwa 15 bis 30°C unter starkem Rühren.

Als wasserlösliche Tenside in der Wasserphase der als Endprodukt durch Vermischen der W/O-Emulsion mit der Wasserphase hergestellten Öl-im-Wasser-Emulsion werden anionische, amphotere bzw. zwitterionische und/oder kationische sowie gegebenenfalls auch spezielle nichtionische Tenside eingesetzt. Sie sind vorzugsweise in einer Menge von etwa 0,25 bis etwa 5 Gew.-%, insbesondere etwa 0,4 bis 2,5 Gew.-% der Gesamtzusammensetzung, berechnet auf die einsatzfertige Öl-in-Wasser- Emulsion, enthalten.

Geeignete anionische Tenside sind solche vom Sulfat-, Sulfonat-, Carboxylat- und Alkylphosphat-Typ, vor allem natürlich diejenigen, die in Haarbehandlungsmitteln üblicherweise zum Einsatz gelangen, insbesondere die bekannten C₁₀-C₁₈-Alkylsulfate und die entsprechenden Ethersulfate, beispielsweise C₁₂-C₁₄-Alkylethersulfat, Laurylethersulfat, insbesondere mit 1 bis 4 Ethylenoxidgruppen im Molekül, Acylaminocarbonsäuren wie Lauroylsarkosinat und -glutamat, weiterhin Monoglycerid(ether)sulfate, Fettsäureamidsulfate, die durch Ethoxylierung und anschließende Sulfatierung von Fettsäurealkanolamiden erhalten werden, und deren Alkalisalze, sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und Alkalisalze von Sulfobernsteinsäurehalbestern, beispielsweise das Dinatriumsalz des Monooctylsulfosuccinats, und Alkalisalze langkettiger Monoalkylethoxysulfosuccinate.
Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 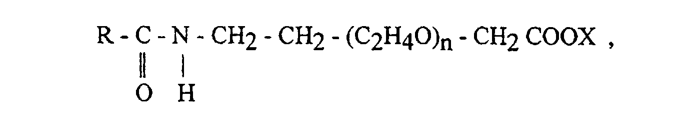 worin R und X die vorstehend angegebene Bedeutung haben und n insbesondere für eine Zahl von 1 bis 10, vorzugsweise 2,5 bis 5, steht.
Derartige Produkte sind seit längerem bekannt und im Handel, beispielsweise unter den Handelsnamen "AKYPO®" und "AKYPO-SOFT®".

Auch C₈-C₂₀-Acylisethionate können, allein oder im Gemisch mit anderen Tensiden, eingesetzt werden, ebenso Sulfofettsäuren und deren Ester.

Es können auch amphotere bzw. zwitterionische Tenside als wasserlösliche Emulgatoren verwendet werden, insbesondere auch im Gemisch mit anionaktiven Tensiden.
Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen können Betaine der Struktur 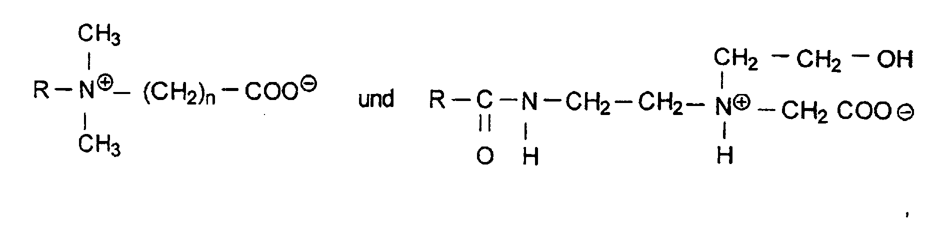 wobei R eine C₈-C₁₈-Alkylgruppe und n 1 bis 3 bedeuten,
Sulfobetaine der Struktur 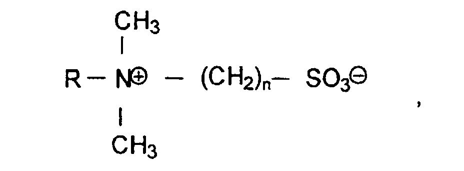 wobei R eine C₈-C₁₈-Alkylgruppe und n 1 bis 3 bedeuten,
und Amidoalkylbetaine der Struktur 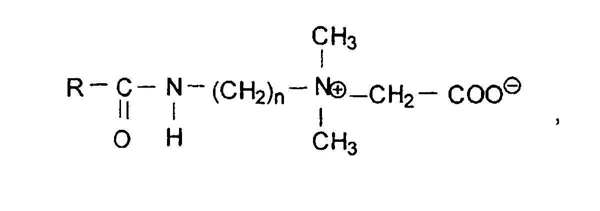 wobei R eine C₈-C₁₈-Alkylgruppe und n 1 bis 3 bedeuten, eingesetzt werden.

Auch die Verwendung geringer Mengen nichtionischer wasserlöslicher Tenside, z.B. von C₈-C₁₈-Alkylpolyglucosiden mit einem Polymerisationsgrad von 1 bis 5, insbesondere im Gemisch mit anionischen und/oder amphoteren bzw. zwitterionischen oberflächenaktiven Substanzen ist möglich.

Weitere geeignete Tenside sind auch kationische Tenside wie die bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 5, vorzugsweise 0,25 bis 5, besonders bevorzugt 0,5 bis 2,5 Gew.-%, der Gesamtzusammensetzung, allein oder vorzugsweise im Gemisch mit amphoteren bzw. zwitterionischen oder gegebenenfalls nichtionischen Tensiden.

Geeignete langkettige quatemäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumbromid, Behenyltrimoniumchlorid, Stearyltrimethylammoniumchlorid, Hydroxyethylhydroxycetyldimoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, etc. Grundsätzlich sind alle quatemären Ammoniurnverbindungen geeignet, die im CTFA International Cosmetic Ingredient Dictionary unter der Bezeichnung " Quaternium" aufgeführt sind.

Die erfindungsgemäß hergestellte Haarfärbeemulsion enthält mindestens ein Oxidationsfarbstoffvorprodukt, zweckmäßigerweise ein Gemisch aus mindestens einer Entwickler- und mindestens einer Kupplersubstanz.

Diese sind an sich bekannt und beispielsweise in der Monographie von K. Schrader, Grundlagen und Rezepturen der Kosmetika, 2. Aufl. (1989), S. 784-799, beschrieben.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2'Hydroxyethylamino)-5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol,4-(N-methyl)-aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, -Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in der erfindungsgemäß hergestellten Farbemulsion kann jeweils etwa 0,1 bis etwa 5 Gew.-%, je nach gewünschter Färbung, betragen.

Diese Oxidationsfarbstoffvorprodukte sind zweckmäßigerweise bereits in der wäßrigen Phase enthalten; sie können jedoch auch, falls erwünscht, dem Fertigprodukt zusammen mit der Wasser-in-Öl-Emulsion oder im Anschluß daran zugesetzt werden.

Die erfindungsgemäß hergestellten Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die Herstellung der endgültigen Öl-in-Wasser-Emulsion erfolgt durch Einrühren der Wasser-in-Öl-Emulsion in die wäßrige Phase bei etwa 15 bis 30°C, insbesondere Raumtemperatur, d.h. bei etwa 20 bis 25°C, bei etwa 5000 bis 15 000, insbesondere 8000 bis 12 000 U/min.

Die erfindungsgemäßen Haarfärbemittel-Emulsionen können die in solchen Mitteln üblichen Grund- und Zusatzstoffe, Konditioniermittel, Stabilisatoren, Verdickungsmittel, Komplexbildner, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Die erfindungsgemäß hergestellten Färbeemulsionen weisen vorzugsweise einen im alkalischen Bereich liegenden pH-Wert, insbesondere zwischen etwa 8 und etwa 12,5, vorzugsweise zwischen 8,5 und 11, auf, der insbesondere durch Zusatz von Ammoniak eingestellt wird.

Die Viskosität der gebrauchsfertigen Öl-in-Wasser-Emulsion liegt vorzugsweise bei etwa 5000 bis 30 000, insbesondere etwa 7 500 bis 25 000, vorzugsweise etwa 10 000 bis 20 000 mPa.s, gemessen bei 20°C im Brookfield-Viskosimeter RVT.

Zur Applikation wird das erfindungsgemäß hergestellte Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemitteln, d.h. nach Vermischung mit Peroxid, kann sowohl im schwach sauren, d.h. einem Bereich von 5,5 bis 6,9, im neutralen als auch im alkalischen Bereich, d.h. zwischen pH 7,1 und 10 liegen.

Im folgenden werden zwei Ausführungsbeispiele zur Erläuterung der Erfindung gegeben

Es wurden, durch Vermischen der Fettphasenkomponenten bei etwa 60°C und nachfolgende Zugabe von Wasser unter starkem Rühren und Abkühlung auf etwa 25°C, zunächst die folgenden Wasser-in-Öl-Emulsionen hergestellt.

| **Zusammensetzungen** | **Nr. 1** | **Nr. 2** | **Nr. 3** | **Nr. 4** |
|---|---|---|---|---|
| Cetearylalkohol | 32 | - | - | 32 |
| Stearinsäuremonoethanolamid | - | 32 | - | - |
| Ethandioldistearat | - | - | 32 | - |
| Ölsäure | 16 | 16 | 16 | 16 |
| Oleth-5 | 32 | 22 | 32 | - |
| Oleylalkohol | 10 | 10 | 10 | 10 |
| Laureth-12 | - | 10 | - | - |
| Laurylpolyglucosid (P.D.∼1,5) | - | - | - | 22 |
| Wasser ad | 100 | 100 | 100 | 100 Gew.-% |

30 Gew.-% der jeweiligen Wasser-in-Öl-Emulsion Nr. 1,2,3 oder 4 wurden unter Rühren bei 9000 U/min bei 20 bis 25°C in zwei unterschiedliche wäßrige Tensidzusammensetzungen eingebracht:

| **Zusammensetzung** | **Nr. I** | **Nr. II** |
|---|---|---|
| W/O-Emulsionen nach den Beispielen 1,2,3 oder 4 | 30 | 30 |
| Hydroxycetylhydroxyethyldimoniumchlorid | 0,5 | - |
| Natriumlaurylsulfat | - | 0,5 |
| Ammoniak ad pH | 8,5 | 10,5 |
| Wasser ad | 100,0 | 100,0 Gew.-% |

Viskosität: Etwa 15 000 bis 20 000 mPa.s, gemessen im Brookfield-Viskosimeter RVT bei 20°C.
Die erhaltenen Emulsionen sind lagerstabil und können unter Zusatz von wäßrigen Oxidationsfarbstoff-Vorprodukt-Zusammensetzungen, die gegebenenfalls weitere Zusatzstoffe enthalten, zu mit Peroxiden mischbaren Haarfärbeemulsionen konfektioniert werden.

## Patentansprüche

1. Verfahren zur Herstellung von stabilen wäßrigen Haarfärbeemulsionen, enthaltend mindestens ein Oxidationsfarbstoff-Vorprodukt, dadurch gekennzeichnet, daß eine Wasser-in-Öl-Emulsion (A), enthaltend a) 10 bis 50 Gew.-% mindestens eines nichtionischen Emulgators, b) 10 bis 50 Gew.-% mindestens eines nichtionischen Co-Emulgators, ausgewählt aus der Gruppe C₁₀-C₂₂-Fettalkohole, C₁₂-C₁₈-Fettsäuremono- und dialkanolamide und/oder C₁₀-C₂₂-Fettsäureestern mit mehrwertigen Alkoholen, c) 5 bis 40 Gew.-% Ölsäure, und d) bis zu 25 Gew.-% Wasser, jeweils berechnet auf die Gesamtzusammensetzung der W/O-Emulsion, mit einer wäßrigen Phase (B), die mindestens ein wasserlösliches Tensid enthält, bei 15 bis 30°C unter Scherkraft gemischt wird.

2. Verfahren nach Anspruch 1, dadurch gekennzeichnet, daß das Oxidationsfarbstoffvorprodukt in der wäßrigen Phase (B) enthalten ist.

3. Verfahren nach Anspruch 1, dadurch gekennzeichnet, daß das Oxidationsfarbstoffvorprodukt der nach dem Vermischen der W/O-Emulsion (A) und der wäßrigen Phase (B) erhaltenen Öl-in-Wasser-Emulsion zugesetzt wird.

4. Verfahren nach einem oder mehreren der Ansprüche 1 bis 3, dadurch gekennzeichnet, daß die W/O-Emulsion (A) etwa 5 bis 15 Gew.-%, berechnet auf deren Zusammensetzung, Oleylalkohol enthält.

5. Verfahren nach einem oder mehreren der Ansprüche 1 bis 4, dadurch gekennzeichnet, daß die durch Vermischen der W/O-Emulsion (A) mit der wäßrigen Phase (B) erhaltene Öl-in-Wasser-Emulsion eine pH-Wert von etwa 8 und etwa 12,5 aufweist.

6. Verfahren nach einem oder mehreren der Ansprüche 1 bis 5, dadurch gekennzeichnet, daß die durch Vermischen der W/O-Emulsion (A) mit der wäßrigen Phase (B) erhaltene Öl-in-Wasser-Emulsion eine Viskosität von etwa 5 000 bis 30 000 mPa.s, gemessen im Brookfield-Viskosimeter RVT bei 20°C, aufweist.

## Claims

1. Process for the preparation of stable, aqueous hair dyeing emulsions, comprising at least one oxidation dyestuff precursor, whereby a water-in-oil emulsion (A), comprising
a) 10 % to 50 % by weight, of at least one nonionic emulsifier,
b) 10 % to 50 % by weight, of at least one nonionic co-emulsifier selected from the group of C₁₀-C₂₂-fatty alcohols, C₁₂-C₁₈-fatty acid mono- and dialkanolamides and/or C₁₀-C₂₂-fatty acid esters with polyvalent alcohols,
c) 5 % to 40 % by weight of oleic acid, and
d) up to 25 % of water, each calculated to the total W/0-emulsion composition, is mixed with an aqueous phase (B), comprising at least one water-soluble surfactant, under shear force at 15° to 30°C.

2. Process according to claim 1, wherein the oxidation dyestuff precursor is contained in the aqueous phase (B).

3. Process according to claim 1, wherein the oxidation dyestuff precursor is added to the oil-in-water emulsion obtained after admixture of the w/o emulsion (A) with the aqueous phase (B).

4. Process according to one or more of claims 1 to 3, wherein the w/o emulsion (A) comprises about 5 % to 15 % by weight, calculated to the composition thereof, of oleyl alcohol.

5. Process according to one or more of claims 1 to 4, wherein the oil-in-water emulsion obtained by admixture of the w/o emulsion (A) with the aqueous phase (B), has a pH-value of about 8 and about 12.5.

6. Process according to one or more of claims 1 to 5, wherein the oil-in-water emulsion obtained by admixture of the w/o emulsion (A) with the aqueous phase (B) has a viscosity of about 5,000 to 30,000 mPa.s, measured in a Brookfield Viscosimeter RVT at 20°C.

## Revendications

1. Procédé de préparation d'émulsions de teinture pour cheveux aqueuses et stables, contenant au moins un précurseur de colorant d'oxydation, caractérisé en ce que l'on mélange, sous l'effet d'une force de cisaillement et à une température comprise entre 15 et 30 °C, une émulsion eau dans huile (A) contenant a) 10 à 50 % en poids d'au moins un émulsifiant non ionique, b) 10 à 50 % en poids d'au moins un co-émulsifiant non ionique choisi dans le groupe constitué d'alcools gras en C₁₀ à C₂₂, de mono- et dialcanolamides d'acides gras en C₁₂ à C₁₈, et/ou d'esters d'acides gras en C₁₀ à C₂₂ avec des alcools polyvalents, c) 5 à 40 % en poids d'acide oléique et d) jusqu'à 25 % en poids d'eau, chaque fois respectivement rapporté à la composition totale de l'émulsion E/H, avec une phase aqueuse (B) qui contient au moins un tensioactif soluble dans l'eau.

2. Procédé selon la revendication 1, caractérisé en ce que la précurseur de colorant d'oxydation est contenu dans la phase aqueuse (B).

3. Procédé selon la revendication 1, caractérisé en ce que le précurseur de colorant d'oxydation est ajouté à l'émulsion huile dans eau obtenue après avoir mélangé l'émulsion E/H (A) et la phase aqueuse (B).

4. Procédé selon une ou plusieurs des revendications 1 à 3, caractérisé en ce que l'émulsion E/H (A) contient, sur la base de sa composition, environ 5 à 15 % en poids d'alcool oléylique.

5. Procédé selon une ou plusieurs des revendications 1 à 4, caractérisé en ce que l'émulsion huile dans eau obtenue en ayant mélangé l'émulsion E/H (A) avec la phase aqueuse (B) présente un pH compris entre environ 8 et environ 12,5.

6. Procédé selon une ou plusieurs des revendications 1 à 5, caractérisé en ce que l'émulsion huile dans eau obtenue en ayant mélangé l'émulsion E/H(A) avec la phase aqueuse (B) présente une viscosité comprise entre environ 5 000 et 30 000 mPa.s, telle que déterminée au moyen d'un viscosimètre rotatif Brookfield RVT à 20 °C.
